(19) Europäisches Patentamt / European Patent Office / Office européen des brevets

(11) **EP 4 026 534 A1**

(12) **EUROPEAN PATENT APPLICATION**
published in accordance with Art. 153(4) EPC

(43) Date of publication:
**13.07.2022 Bulletin 2022/28**

(21) Application number: **20859738.5**

(22) Date of filing: **31.08.2020**

(51) International Patent Classification (IPC):
*A61K 8/34* (2006.01)   *A61K 8/26* (2006.01)
*A61K 8/41* (2006.01)   *A61K 8/44* (2006.01)
*A61K 8/46* (2006.01)   *A61K 8/60* (2006.01)
*A61K 47/02* (2006.01)   *A61K 47/10* (2017.01)
*A61K 47/18* (2017.01)   *A61K 47/20* (2006.01)
*A61K 47/26* (2006.01)   *A61Q 15/00* (2006.01)

(52) Cooperative Patent Classification (CPC):
A61K 8/26; A61K 8/34; A61K 8/41; A61K 8/44;
A61K 8/46; A61K 8/60; A61K 47/02; A61K 47/10;
A61K 47/18; A61K 47/20; A61K 47/26; A61Q 15/00

(86) International application number:
**PCT/JP2020/032897**

(87) International publication number:
**WO 2021/045005 (11.03.2021 Gazette 2021/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **06.09.2019 JP 2019162740**

(71) Applicant: **Rohto Pharmaceutical Co., Ltd.
Osaka-shi, Osaka 544-8666 (JP)**

(72) Inventor: **INOUE Shota
Osaka-shi, Osaka 544-8666 (JP)**

(74) Representative: **Morf, Jan Stefan et al
Abitz & Partner
Patentanwälte mbB
Arabellastrasse 17
81925 München (DE)**

(54) **COMPOSITION FOR EXTERNAL APPLICATION**

(57)    An object of the invention is to provide a topical composition excellent in adhesiveness with respect to the skin.

The topical composition comprises (A) at least one polyhydric alcohol having a molecular weight of 100 to 200, and (B) at least one non-polymerizable cationic compound. The component (B) may be a compound containing a metal to be a divalent or higher cation in an aqueous solution, a basic amino acid, alkanol amine, or alkyl amine, and the component (A) may be glucose, sorbitol, fructose, xylitol, or erythritol.

**Description**

TECHNICAL FIELD

The present invention relates to a topical composition(an external preparation). More specifically, the invention relates to a topical composition excellent in adhesiveness with respect to the skin.

BACKGROUND ART

An antiperspirant is used by being applied onto the skin. In general, the region of the body to which the antiperspirant is applied is a region such as the armpits or the back, in which friction occurs due to contact with other regions of the body or clothes. Accordingly, there is a problem that the formulation is peeled off from the application region by the friction, and thus, a formulation having higher adhesiveness with respect to the application region has been required to be developed.

In Patent Document 1, an antiperspirant composition smoothly spreading at the time of application is disclosed in which a resin adheres to the skin even after the application, and smoothness is maintained. Such a composition contains an antibromic component and specific spherical resin particles. In the antiperspirant composition of Patent Document 1, not the formulation itself containing the antibromic component but the resin particles adhere to the skin.

On the other hand, in Patent Document 2, an antiperspirant composition that does not cause the tenseness and the stickiness of the skin after application, is excellent in an antiperspirant effect and formulation stability, and is capable of suppressing the darkening of the armpits is disclosed. Such a composition contains (A) an antiperspirant component such as an aluminum compound, (B) specific polyoxypropylene stearyl ether, (C) an anti-inflammatory component, (D) at least one type selected from trimethyl glycine, sodium pyrrolidone carboxylate, sorbitol, serine, and glycine, (E1)/(E2) specific polyoxyethylene stearyl ether, and (F) water. In the antiperspirant composition of Patent Document 2, the adhesiveness with respect to the skin is not considered.

CITATION LIST

PATENT DOCUMENT

Patent Document 1: JP 2004-059501 A
Patent Document 2: JP 2018-203683 A

SUMMARY OF THE INVENTION

PROBLEM TO BE SOLVED BY THE INVENTION

An object of the invention is to provide a topical composition excellent in adhesiveness with respect to the skin.

MEANS FOR SOLVING PROBLEM

As a result of intensive studies of the present inventors in order to attain the object described above, it has been found that a topical composition excellent in adhesiveness with respect to the skin can be provided by using polyhydric alcohol having a specific molecular weight and a cationic non-polymerizable compound together, and the invention has been completed.

That is, the invention relates to

[1] a topical composition, comprising:

(A) at least one polyhydric alcohol having a molecular weight of 100 to 200; and
(B) at least one non-polymerizable cationic compound.

[2] It is preferable that in the composition according to [1], the number of hydroxy groups of the component (A) is 4 or more.
[3] It is preferable that in the composition according to [1] or [2], the component (B) is one or more types selected from the group consisting of compound containing a metal to be a divalent or higher cation in an aqueous solution, a basic amino acid, alkanol amine, and alkyl amine.

[4] It is preferable that in the composition according to any one of [1] to [3], the component (A) is one or more types selected from the group consisting of glucose, sorbitol, fructose, xylitol, and erythritol.

[5] It is preferable that in the composition according to any one of [1] to [4], the component (B) is one or more types selected from the group consisting of zinc para-phenolsulfonate, aluminum chlorohydrate(chlorohydroxyaluminum), alum, arginine, and lysine.

[6] It is preferable that in the composition according to any one of [1] to [5], a concentration of the component (A) is 4 to 20 wt.%.

[7] The topical composition according to any one of [1] to [6] is suitable for being used as an antiperspirant composition.

[8] It is preferable that in the composition according to any one of [1] to [7], when a total amount of the component (A) is set to 1 part by weight, a total amount of the component (B) is 0.01 to 25 parts by weight.

[9] Examples of one preferred embodiment of the composition according to [1] include antiperspirant composition, comprising:

(A) 1 to 20 wt.% of at least one polyhydric alcohol selected from the group consisting of xylitol and erythritol; and
(B) 1 to 20 wt.% of at least one non-polymerizable cationic compound selected from the group consisting of zinc para-phenolsulfonate, aluminum chlorohydrate, and alum.

[10] Examples of one preferred embodiment of the composition according to [1] include an antiperspirant composition, comprising:

(A) 1 to 15 wt.% of erythritol; and
(B) 1 to 20 wt.% of at least one non-polymerizable cationic compound selected from the group consisting of zinc para-phenolsulfonate and aluminum chlorohydrate;

in which when a total amount of the component (A) is set to 1 part by weight, a total amount of the component (B) is in a range of 1 to 3 parts by weight.

[11] The composition according to any one of [1] to [10] has film-forming ability.

[12] In addition, the invention relates to use of (A) at least one polyhydric alcohol having a molecular weight of 100 to 200 for reacting with (B) at least one non-polymerizable cationic compound to form a film that adheres to skin.

In the use, preferred examples, concentrations, parts by weight, combinations, and applications of the component (A) and the component (B) are as described in [2] to [10].

EFFECT OF THE INVENTION

According to the invention, it is possible to provide a topical composition excellent in adhesiveness with respect to the skin.

BRIEF DESCRIPTION OF DRAWINGS

Fig. 1 is a picture illustrating a device and a test method that are used in an adhesiveness (film strength) evaluation test of Test Example 1; and
Fig. 2 is a picture illustrating a test method of an astringency evaluation test of Test Example 2.

MODE(S) FOR CARRYING OUT THE INVENTION

A composition according to the invention contains (A) at least one polyhydric alcohol having a molecular weight of 100 to 200 and (B) at least one non-polymerizable cationic compound.

According to the invention, a formulation having high adhesiveness with respect to the skin can be obtained by blending both of the component (A) and the component (B). The mechanism by which the composition exhibits excellent skin adhesiveness is not obvious, but there is a possibility in which the component (A) and the component (B) form a film on the skin to adhere to the skin by an interaction between a hydroxy group derived from the component (A) and the cation of the component (B).

In general, a composition for forming a film on the skin contains a polymer as a film forming component. In contrast, the composition of the invention contains not the polymer but the polyhydric alcohol of the component (A) and the cationic compound (a non-polymer) of the component (B). Both of the component (A) and the component (B) have a low molecular weight, but are capable of forming a film on the skin, and thus, exhibit high adhesiveness with respect to the skin.

The component (A) used for the topical composition is polyhydric alcohol having a molecular weight in a range of 100 to 200 (that is, a molar mass of 100 to 200 g/mol).

It is preferable that the polyhydric alcohol has 2 to 9 hydroxy groups, from the viewpoint of further increasing the effect of the invention. The number of hydroxy groups is more preferably 3 to 6, and particularly preferably 4 to 5.

It is preferable that the component (A) used for the topical composition is polyhydric alcohol having a linear carbon chain. The mechanism is unobvious, but this is because in a case where the polyhydric alcohol has a linear carbon chain, a film is likely to be formed.

From the viewpoint of further increasing the effect of the invention, examples of the preferred component (A) include sugars having a molecular weight of 100 to 200 and hydroxy groups, and in particular, monosaccharide or monosaccharide alcohol (sugar alcohol) is more preferable. Preferred examples of the component (A) include glucose (a molecular weight of approximately 180), sorbitol (a molecular weight of approximately 182), fructose (a molecular weight of approximately 180), xylitol (a molecular weight of approximately 152), erythritol (a molecular weight of approximately 122), 1,6-hexanediol (a molecular weight of approximately 118), 1,8-octanediol (a molecular weight of approximately 146), 1,9-nonanediol (a molecular weight of approximately 160), 1,10-decanediol (a molecular weight of approximately 174), 1,11-undecanediol (a molecular weight of approximately 188), and 1,2-decanediol (a molecular weight of approximately 174). The component (A) is particularly preferably erythritol, xylitol, and sorbitol, even more preferably erythritol and xylitol, and most preferably erythritol. The component (A) is generally commercially available, and thus, is easily available.

The molecular weight of the component (A) used for the topical composition is not particularly limited, and is preferably 100 to 200, more preferably 110 to 190, and even more preferably 120 to 160. It is difficult to form a film with polyhydric alcohols having a molecular weight of less than 100, probably because they are often a liquid at a normal temperature. Therefore, such polyhydric alcohols are not preferable. Probably because polyhydric alcohol having a molecular weight of greater than 200 has excessively high crystallizability, a film derived therefrom has low adhesiveness with respect to the skin. Therefore, such polyhydric alcohols are not preferable. It is preferable that the component (A) used for the topical composition is a solid at a normal temperature (for example, 15 to 25°C).

In the topical composition, only one type of the components (A) described above may be used, or two or more types thereof may be used in combination.

The content of the component (A) is not particularly limited, and is preferably 0.01 to 25 wt.% (w/w%), more preferably 1 to 20 wt.%, even more preferably 3 to 15 wt.%, particularly preferably 5 to 12 wt.%, and most preferably 7 to 10 wt.%, with respect to the entire composition, from the viewpoint of further increasing the effect of the invention.

The component (B) used for the topical composition is a non-polymerizable cationic compound. Herein, the "non-polymerizable" compound indicates that the compound is not a polymer formed by polymerizing a monomer. In addition, the "non-polymerizable" compound according to the invention is not a monomer for forming a polymer by being polymerized. It is preferable that the component (B) is a low-molecular compound having a molecular weight of 500 or less. Note that, in a case where the component (B) is a hydrate, the molecular weight indicates a molecular weight excluding $H_2O$. Specific examples of the component (B) include a compound containing a metal to be a divalent or higher cation in an aqueous solution, a basic amino acid, alkanol amine, and alkyl amine.

In a case where the component (B) is the compound containing a metal to be a divalent or higher cation in an aqueous solution, specific examples thereof include an aluminum-based compound (that is, an aluminum-based compound that generates trivalent cations [$Al^{3+}$] in an aqueous solution) such as aluminum chlorohydrate, aluminum chloride, aluminum sulfate, bromohydroxyaluminum, and aluminum potassium sulfate hydrate (a kind of alum), and a zinc-based compound (that is, a zinc-based compound that generates divalent cations [$Zn^{2+}$] in an aqueous solution) such as zinc para-phenolsulfonate. All of the compounds have an antiperspirant action and are known as so-called aluminum-based antiperspirant components, zinc-based antiperspirant components, and the like. By using the antiperspirant component as the component (B), the antiperspirant component and the polyhydric alcohol of the component (A) form a film on the skin to adhere to the skin. Accordingly, according to the invention, it is possible to allow the antiperspirant component itself to directly adhere to the skin. That is, the invention can provide an antiperspirant composition that is less likely to be removed from the skin and has a high antiperspirant effect.

Examples of a case in which the component (B) is the basic amino acid may include arginine, lysine, histidine, triptophan, and ornithine. Such basic amino acids are preferable since they are known to have an antibacterial action.

Examples of a case in which the component (B) is the alkanol amine include ethanol amine, diethanol amine, triethanol amine, isopropanol amine, diisopropanol amine, triisopropanol amine, methyl ethanol amine, methyl diethanol amine, 2-amino-2-hydroxymethyl-1,3-propanediol, 2-amino-2-methyl-1-propanol, and 2-amino-2-methyl-1,3-propanediol. Such alkanol amines are preferable since they are known to have an antibacterial action.

Examples of a case in which the component (B) is the alkyl amine include cetyl trimethyl ammonium bromide, cetyl pyridinium bromide, and benzalkonium chloride. Such alkyl amines are preferable since they are known to have an antibacterial action.

By using the basic amino acid, the alkanol amine, or the alkyl amine, which are known to have the antibacterial action, as the component (B), the component (B) having an antibacterial action and the polyhydric alcohol of the component (A) form a film on the skin to adhere to the skin. Accordingly, according to the invention, it is possible to allow the antiperspirant component itself to directly adhere to the skin. That is, the invention can provide a composition that is less likely to be removed from the skin and has a high antibacterial effect. Such a composition is useful as a deodorant.

The particularly preferred component (B) is the compound containing a metal to be a divalent or higher cation in the aqueous solution. Among them, aluminum chlorohydrate, zinc para-phenolsulfonate, and alum (for example, $AlK(SO_4)_2 \cdot 12H_2O$) are more preferable, aluminum chlorohydrate and zinc para-phenolsulfonate are particularly preferable, and aluminum chlorohydrate is even more preferable.

In the topical composition, only one type of the components (B) described above may be used, or two or more types thereof may be used in combination.

The content of the component (B) is not particularly limited, and is preferably 0.1 to 20 wt.% (w/w%), more preferably 0.3 to 15 wt.%, even more preferably 1 to 12 wt.%, and particularly preferably 5 to 10 wt.%, with respect to the entire composition, from the viewpoint of further increasing the effect of the invention.

In the topical composition, when the total amount of the component (A) is set to 1 part by weight, the total amount of the component (B) is preferably 0.01 to 25 parts by weight, more preferably 0.1 to 8 parts by weight, even more preferably 0.375 to 5 parts by weight, still even more preferably 1 to 3 parts by weight, and particularly preferably 1.25 to 1.5 parts by weight, from the viewpoint of further increasing the effect of the invention.

The topical composition is particularly suitable for being used as a formulation required to have improved adhesiveness with respect to the skin, for example, an antiperspirant or a deodorant, and in particular, is suitable for being used as an antiperspirant.

The invention can be used for the topical composition to adhere to a desired region of the skin, and can also be used for the topical composition to adhere to the region of the skin for a long period of time. By improving the adhesiveness, it is possible to provide a topical composition that is resistant to friction. In particular, by allowing the antibacterial component to adhere to the skin, it is possible to retain an application region clean or suppress a body odor. Accordingly, in order to retain the application region clean or suppress the odor of the application region, the topical composition may be used.

The topical composition may further contain a cooling agent (refreshing agent) in addition to the component (A) and the component (B). The cooling agent is not particularly limited, and for example, menthol, menthyl glyceryl ether, menthyl lactate, mint oil, peppermint oil, camphor, icilin, and the like can be used, and among them, menthol is preferable. Only one type of the cooling agents may be used, or two or more types thereof may be used together.

In a case where the cooling agent is blended in the topical composition, the content thereof is not particularly limited, and is preferably 0.001 to 10 wt.%, more preferably 0.01 to 5 wt.%, and even more preferably 0.1 to 1 wt.%, with respect to the entire composition.

The topical composition may further contain a surfactant in addition to the component (A) and the component (B).

The surfactant is not particularly limited, and for example, various non-ionic surfactants can be exemplified, such as alkyl glyceryl ether such as isostearyl glyceryl ether (monoisostearyl glyceryl ether, diisostearyl glyceryl ether, and the like), myristyl glyceryl ether (monomyristyl glyceryl ether, dimyristyl glyceryl ether, and the like), and lauryl glyceryl ether (monolauryl glyceryl ether, dilauryl glyceryl ether, and the like); polyoxyethylene (hereinafter, may be referred to as POE)-branched alkyl ether such as POE-octyl dodecyl alcohol, and POE-2-decyl tetradecyl alcohol; POE-alkyl ether such as POE-oleyl alcohol ether and POE-cetyl alcohol ether; sorbitan ester such as sorbitan monooleate, sorbitan monoisostearate, and sorbitan monolaurate; POE-sorbitan ester such as POE-sorbitan monooleate, POE-sorbitan monoisostearate, and POE-sorbitan monolaurate; glycerin fatty acid ester such as glyceryl monooleate, glyceryl monostearate, and glyceryl monomyristate; POE-glycerin fatty acid ester such as POE-glyceryl monooleate, POE-glyceryl monostearate, and POE-glyceryl monomyristate; POE-dihydrocholesterol ester, POE-hardened castor oil, and POE-hardened castor oil fatty acid ester such as POE-hardened castor oil isostearate; POE-alkyl aryl ether such as POE-octyl phenyl ether; POE-alkyl glyceryl ether such as POE-monostearyl glyceryl ether and POE-monomyristyl glyceryl ether; polyoxyethylene polyoxypropylene alkyl ether (hereinafter, polyoxypropylene may be referred to as POP) such as polypropylene glycol (hereinafter, may be referred to as PPG)-4-ceteth-1, PPG-6-decyl tetradeces-20, and POE POP butyl ether; POP polyglyceryl ether such as POP diglyceryl ether; POE-POP glyceryl ether; and polyglycerol fatty acid ester such as diglyceryl monostearate, decaglyceryl decastearate, decaglyceryl decaisostearate, diglyceryl diisostearate, and polyglyceryl monolaurate. Alternatively, a naturally derived surfactant such as lecitin, hydrogenated lecitin, saponin, sodium surfactin, cholesterol, and a bile acid, and the like can be exemplified. The preferred surfactant is the non-ionic surfactant. In the topical composition, only one type of the surfactants described above may be used, or two or more types thereof may be used in combination.

In a case where the surfactant is blended in the topical composition, the content thereof is not particularly limited, and is preferably 0.1 to 20 wt.%, more preferably 0.5 to 10 wt.%, and even more preferably 1 to 5 wt.%, with respect to the entire composition, from the viewpoint of further increasing the effect of the invention.

The topical composition may further contain lower alcohol in addition to the component (A) and the component (B). Herein, the lower alcohol indicates monohydric alcohol having 1 to 6 carbon atoms. The lower alcohol is not particularly limited insofar as the lower alcohol is pharmaceutically or physiologically acceptable. Specifically, examples of the lower alcohol include ethanol, propanol, isopropanol, n-butyl alcohol, s-butyl alcohol, t-butyl alcohol, isobutyl alcohol, pentyl alcohol, and hexyl alcohol, but the lower alcohol is not limited thereto. From the viewpoint of more effectively exhibiting the effect of the invention, ethanol, propanol, isopropanol, n-butyl alcohol, s-butyl alcohol, t-butyl alcohol, and isobutyl alcohol are preferable, ethanol and isopropanol are more preferable, and ethanol is even more preferable. In the topical composition, only one type of the lower alcohols described above may be used, or two or more types thereof may be used in combination.

In a case where the lower alcohol is blended in the topical composition, the content thereof is not particularly limited, and is preferably 0.1 to 95 wt.% (for example, 1 to 60 wt.%), 5 to 80 wt.% (for example, 5 to 50 wt.%), more preferably 7 to 75 wt.% (for example, 7 to 40 wt.%), and particularly preferably 8 to 10 wt.%, with respect to the entire composition, from the viewpoint of further increasing the effect of the invention.

The topical composition may further contain an oiling agent in addition to the component (A) and the component (B). The oiling agent is not particularly limited, and examples thereof include hydrocarbon, oils and fats, waxes, ester oil, higher alcohol, a higher fatty acid, silicone oil, and the like.

Examples of the hydrocarbon include paraffin, ceresin, isoparaffin, hard fat, microcrystalline wax, polybutene, a polyethylene powder, liquid paraffin, squalane, petroleum jelly(vaseline), gelled hydrocarbon (Plastibase or the like), ozokerite, α-olefin oligomer, light liquid paraffin, and the like.

Examples of the oils and fats include hardened oil such as hydrogenated palm oil and hydrogenated rice bran oil; avocado oil, linseed oil, camellia oil, macadamia nut oil, corn oil, olive oil, safflower oil, apricot kernel oil, cinnamon oil, jojoba oil, grapeseed oil, sunflower seed oil, almond oil, camellia oleifera seed oil, canola oil, sesame oil, cacao butter, coconut oil, hardened coconut oil, palm oil, palm kernel oil, Japan wax kernel oil, Japan wax, wheat germ oil, rice germ oil, rice bran oil, cottonseed oil, soybean oil, peanut oil, gold tea oil, evening primrose oil, candlenut oil, hazelnut oil, shea butter, and the like.

Examples of the waxes include waxes such as candelilla wax, rice bran wax, cotton wax, carnauba wax, lanolin, lanolin fatty acid isopropyl ester, POE lanolin alcohol ether, POE lanolin alcohol acetate, lanolin fatty acid polyethylene glycol ester, POE hydrogenated lanolin alcohol ether, shellac wax, and beeswax.

Examples of the ester oil include ester oil such as isopropyl myristate, cetyl octanoate, octyl dodecyl myristate, isopropyl palmitate, isopropyl isostearate, octyl palmitate, octyl isopalmitate, cetyl palmitate, butyl stearate, hexyl laurate, myristyl myristate, decyl oleate, isodecyl oleate, hexyl decyl dimethyl octanoate, diisopropyl sebacate, di-2-ethyl hexyl sebacate, 2-hexyl decyl myristate, 2-hexyl decyl palmitate, diisopropyl adipate, 2-hexyl decyl adipate, isononyl isononanoate, isotridecyl isononanoate, cetyl lactate, myristyl lactate, lanolin acetate, isocetyl stearate, isocetyl isostearate, isostearyl isostearate, 12-hydroxycholesteryl stearylate, cholesteryl stearate, cholesteryl oleate, phytosteryl macadamia nut fatty acid ester, phytosteryl oleate, dextrin palmitate, inulin stearate, hydrogenated jojoba oil, ethylene glycol di-2-ethyl hexylate, cetyl 2-ethyl hexanoate, dipentaerythritol fatty acid ester, N-alkyl glycol monoisostearate, neopentyl glycol dicaprate, diisostearyl malate, glyceryl di-2-heptyl undecanoate, tri-2-ethyl hexyl trimellitate, tritridecyl trimellitate, trimethylol propane tri-2-ethyl hexylate, trimethylol propane triisostearate, pentaerythritol tetra-2-ethyl hexanoate, glyceryl tri-2-ethyl hexanoate, trimethylol propane triisostearate, cetyl 2-ethyl hexanoate, glyceryl trimyristate, glyceryl tri(caprylate/caprate), glyceryl tri(caprylate/caprate/myristate/stearate), glyceride tri-2-heptyl undecanoate, castor oil fatty acid methyl ester, oleyl oleate, 2-heptyl undecyl palmitate, diisobutyl adipate, di(phytosteryl/octyl dodecyl) lauroyl glutamate, di(octyl dodecyl/phytosteryl/behenyl) lauroyl glutamate, di-2-heptyl undecyl adipate, ethyl laurate, bisethoxydiglycol cyclohexane-1,4-dicarboxylate, 2-ethyl hexyl succinate, diethoxyethyl succinate, triethyl citrate, decaglyceryl (eicosadioate/tetradecanedioate), ethyl acetate, butyl acetate, amyl acetate, triethyl citrate, (phytosteryl/isostearyl/cetyl/stearyl/behenyl) dimer dilinoleate, polyglyceryl-2 triisostearate, dimer dilinoleyl dimer dilinoleate, dipentaerythrityl tripolyhydroxystearate, glyceryl tri(behenate/isostearate/eicosadioate), polyglyceryl-10 (eicosadioate/tetradecanedioate), bisethoxydiglycol cyclohexane dicarboxylate, bis(triethylene glycol monoethyl ether) 1,4-cyclohexane dicarboxylate, bis(diethylene glycol monoethyl ether) adipate, bis(triethylene glycol monoethyl ether) adipate, diethoxyethyl succinate, and diethyl hexyl succinate.

Examples of the higher alcohol include higher alcohol having 12 to 22 carbon atoms, sterols, and the like. Preferably, saturated linear alcohol (a solid at a normal temperature) such as lauryl alcohol, myristyl alcohol, cetyl alcohol, cetostearyl alcohol, stearyl alcohol, arachidyl alcohol, and behenyl alcohol; unsaturated alcohol (a liquid at a normal temperature) such as oleyl alcohol and selachyl alcohol; branched alcohol (a liquid at a normal temperature) such as hexyl decanol, isostearyl alcohol, octyl dodecanol, decyl tetradecanol, lanolin alcohol, and isostearyl glyceryl ether; and sterols such as phytosterol and cholesterol are exemplified.

Examples of the higher fatty acid include a saturated or unsaturated linear or branched fatty acid having 12 to 22 carbon atoms can be used. For example, preferably, a saturated linear fatty acid (a solid at a normal temperature) such as a lauric acid, a myristic acid, a palmitic acid, a stearic acid, a margaric acid, an arachidonic acid, and a behenic acid; an unsaturated fatty acid (a liquid at a normal temperature) such as an oleic acid, a linoleic acid, a linolenic acid, an arachidonic acid, a palmitoleic acid, an eicosapentaenoic acid, a vaccenic acid, and a docosahexaenoic acid; a branched fatty acid such as a lanolin fatty acid; a 12-hydroxystearic acid, and the like.

As the silicone oil, silicone oil such as siloxane such as methyl polysiloxane, methyl phenyl polysiloxane, decamethyl cyclopentasiloxane, methyl cyclopentasiloxane, high polymeric methyl polysiloxane, octamethyl cyclotetrasiloxane, methyl hydrogen polysiloxane, methyl trimethicone, dimethiconol, and a dimethiconol cross-polymer; alkyl-modified silicone such as caprylyl methicone, amino-modified silicone such as aminopropyl dimethicone and amodimethicone, cross-linked methyl polysiloxane, cross-linked alkyl-modified silicone, amino-modified silicone, polyether-modified silicone, polyglycerin-modified silicone, cross-linked polyether-modified silicone, cross-linked alkyl polyether-modified silicone, silicone-alkyl chain-co-modified polyether-modified silicone, silicone-alkyl chain-co-modified polyglycerin-modified silicone, polyether-modified branched silicone, polyglycerin-modified branched silicone, acrylic silicone, phenyl-modified silicone, and silicone resin are exemplified.

In a case where the oiling agent is blended in the topical composition, the content thereof is not particularly limited, and is preferably 0.01 to 70 wt.%, more preferably 0.1 to 60 wt.%, even more preferably 5 to 50 wt.%, and particularly preferably 10 to 40 wt.%, with respect to the entire composition, from the viewpoint of further increasing the effect of the invention.

In order to produce other useful effects, the topical composition may further contain one type or two or more type of various components (such as a moisturizing component, an antiseptic component, an anti-inflammatory component, a sebum adsorptive component, an ultraviolet scattering component, an ultraviolet absorptive component, a component having an action of preventing and/or repairing DNA damage, a whitening component, a cell activating component, an antioxidant component, an anti-aging component, a keratin softening component, and vitamins) in addition to the components described above. Each of such components is not particularly limited insofar as the components can be used in the field of pharmaceutical products, quasi-pharmaceutical products, cosmetics, or the like, and any of the components can be used by being suitably selected. Specific examples of such components include isopropyl methyl phenol, a glycyrrhetinic acid, a glycyrrhizinic acid and a salt thereof, triclosan, and the like.

The vitamins may be either water-soluble vitamin or oil-soluble vitamin, and examples thereof include vitamin As such as a retinol derivative such as retinol, retinol acetate, retinol palmitate, retinol propionate, and retinol linoleate, hydrogenated retinol, retinal, a retinoic acid, methyl retinoate, ethyl retinoate, retinol retinoate, d-$\delta$-tocopheryl retinoate, $\alpha$-tocopheryl retinoate, and $\beta$-tocopheryl retinoate; provitamin As such as $\beta$-carotene, $\alpha$-carotene, $\gamma$-carotene, $\delta$-carotene, lycopene, zeaxanthin, cryptoxanthin, and echinenone; vitamin Es such as dl-$\alpha$-tocopherol, dl-$\alpha$-tocopherol succinate, calcium dl-$\alpha$-tocopherol succinate, d-$\delta$-tocopherol, tocopherol nicotinate, dl-$\alpha$-tocopherol acetate, tocopherol linoleate, tocopherol (linoleate/oleate), potassium (ascorbyl/tocopheryl) phosphate, and ascorbyl tocopheryl maleate; vitamin B2s such as riboflavine, flavin mononucleotide, flavin adenine dinucleotide, riboflavin butyric ester, riboflavinetetrabutyric ester, sodium riboflavin 5'-phosphoric ester, and riboflavin tetranicotinic ester; nicotinic acids such as methyl nicotinate, a nicotinic acid, benzyl nicotinate, nicotinic-acid amide, $\beta$-butoxyethyl nicotinate, and 1-(4-methyl phenyl) ethyl nicotinate; vitamin Cs such as ascorbyl stearate, 2-sodium isostearyl ascorbyl phosphate, L-ascorbyl dipalmitate, ascorbyl tetraisopalmitate (ascorbyl tetra-2-hexyl decanoate), 3-sodium palmitate ascorbyl phosphate, methyl silanol ascorbate, an ascorbic acid, sodium ascorbate, a dehydroascorbic acid, sodium ascorbic acid phosphate, magnesium ascorbic acid phosphate, 2-sodium ascorbyl sulfate, ascorbic acid glucoside, 2-O-ethyl ascorbate, 3-O-ethyl ascorbate, glyceryl ascorbate, bisglyceryl ascorbate, hexyl 3-glyceryl ascorbate, 3-glyceryl ascorbate, myristyl 3-glyceryl ascorbate, and 3-lauryl glyceryl ascorbate; vitamin Ds such as methyl hesperidin, ergocalciferol, and cholecalciferol; vitamin Ks such as phylloquinone and farnoquinone; vitamin B1s such as thiamine and a salt thereof (for example, dibenzoyl thiamine hydrochloride, thiamine hydrochloride, and thiamine diphosphate); vitamin B6s such as pyridoxine hydrochloride, pyridoxine acetate, pyridoxal hydrochloride, 5'-pyridoxal phosphate, and pyridoxamine hydrochloride; vitamin B12s such as cyanocobalamine, hydroxocobalamin, and dioxyadenosylcobalamin; folic acids such as a folic acid and a pteroylglutamic acid; pantothenic acids such as a pantothenic acid, calcium pantothenate, pantothenyl alcohol (panthenol), D-pantetheine, D-pantethine, coenzyme A, pantothenyl ethyl ether, and calcium pantetein sulfonate; biotins such as biotin and biocytin; and a vitamin-like active factor such as carnitine, a ferulic acid, an $\alpha$-lipoic acid, an orotic acid, $\gamma$-oryzanol, pyrroloquinoline quinone, hesperidin, glucosyl hesperidin, glucuronolactone, glucuronamide, ubiquinone, and salts thereof.

In addition, the topical composition can be prepared by suitably blending components that are generally used in the field of pharmaceutical products, quasi-pharmaceutical products, cosmetics, or the like, in accordance with the use or form, in addition to the components described above. The components to be blended are not particularly limited, and for example, additives such as a base or a carrier, a fragrance, an oxidant inhibitor, a preserving agent, a pH adjuster, a chelating agent, a stabilizing agent, a stimulation reducing agent, an antiseptic agent, a coloring agent, and a dispersant can be blended. Note that, only one type of such components can be blended, or two or more types thereof can be blended in arbitrary combination.

The composition may contain water (purified water). In a case where water is blended in the topical composition, the content thereof is not particularly limited, and is preferably 0.001 to 85 wt.%, and more preferably 10 to 55 wt.%, with respect to the entire composition, from the viewpoint of further increasing the effect of the invention.

It is preferable that the topical composition does not contain liquefied petroleum gas or an isostearic acid. In particular, it is preferable that the topical composition does not contain a combination of liquefied petroleum gas and an isostearic acid.

The topical composition can be prepared in an arbitrary dosage form that is known in the field of pharmaceutical products, quasi-pharmaceutical products, cosmetics, or the like. Specifically, examples thereof include a dosage form suitable for application to the skin, such as a roll-on type, a sheet (indicating a wiping-off type sheet but not a patch type sheet), a cream (preferably, a W/O type), a stick, a lotion, a gel, a spray agent (for example, an aerosolized agent), a spray (a mist spray), and a powder agent, but the dosage form is not limited thereto. It is preferable that the topical composition is in the form of a roll-on type, a sheet, a cream, and a stick, from the viewpoint of more reliably obtaining a higher effect of the invention. Such dosage forms can be prepared by an arbitrary method that is well-known to a person skilled in the art.

As a preferred embodiment of the topical composition, an antiperspirant composition (particularly preferably, an antiperspirant composition in the form of a roll-on type, a sheet, a cream, or a stick), comprising:

(A) 1 to 20 wt.% (more preferably 3 to 15 wt.%, particularly preferably 5 to 12 wt.%, and even more preferably 7 to 10 wt.%) of at least one polyhydric alcohol selected from the group consisting of glucose, sorbitol, fructose, xylitol, and erythritol (more preferably, the group consisting of xylitol and erythritol) (in particular, erythritol); and

(B) 1 to 20 wt.% (more preferably 1 to 15 wt.%, particularly preferably 1 to 12 wt.%, and even more preferably 5 to 10 wt.%) of at least one non-polymerizable cationic compound selected from the group consisting of zinc para-phenolsulfonate, aluminum chlorohydrate, alum, arginine, and lysine (more preferably, the group consisting of zinc para-phenolsulfonate, aluminum chlorohydrate, and alum) (in particular, aluminum chlorohydrate), is exemplified.

It is preferable that in the antiperspirant composition, when the total amount of the component (A) is set to 1 part by weight, the total amount of the component (B) is in a range of 0.1 to 8 parts by weight (more preferably 0.375 to 5 parts by weight, particularly preferably 1 to 3 parts by weight, and even more preferably 1.25 to 1.5 parts by weight). In addition, it is preferable that the antiperspirant composition contains benzalkonium chloride and/or isopropyl methyl phenol in the amount of 0.01 to 0.2 wt.%(more preferably, 0.03 to 0.1 wt.%), respectively. In addition, it is preferable that the antiperspirant composition further contains water (purified water) and/or ethanol (for example, dehydrated ethanol).

As a more preferred embodiment of the topical composition, an antiperspirant composition (particularly preferably, an antiperspirant composition in the form of a roll-on type, a sheet, a cream, or a stick), comprising:

(A) 1 to 15 wt.% (more preferably 3 to 15 wt.%, particularly preferably 5 to 12 wt.%, and even more preferably 7 to 10 wt.%) of erythritol ; and

(B) 1 to 20 wt.% (more preferably 1 to 15 wt.%, particularly preferably 1 to 12 wt.%, and even more preferably 5 to 10 wt.%) of at least one non-polymerizable cationic compound selected from the group consisting of zinc para-phenolsulfonate and aluminum chlorohydrate (in particular, aluminum chlorohydrate),

in which when the total amount of the component (A) is set to 1 part by weight, the total amount of the component (B) is in a range of 1 to 3 parts by weight (more preferably 1.25 to 1.5 parts by weight), is exemplified.

In addition, it is preferable that the antiperspirant composition contains benzalkonium chloride and/or isopropyl methyl phenol (preferably both of benzalkonium chloride and isopropyl methyl phenol) in the amount of 0.01 to 0.2 wt.% (more preferably 0.03 to 0.1 wt.%), respectively. In addition, it is preferable that the antiperspirant composition further contains water (purified water) and/or ethanol (for example, dehydrated ethanol).

A method of using the topical composition may vary depending on the condition of the skin, the age, and the gender of a subject, a situation such as the season to use, or the like. In general, the composition is applied to an arbitrary region of the skin (preferably, the face, behind the ears, the neck, the decollete part, the back, the armpits, the hands, the feet, and the like) in which an antiperspirant action or an antibacterial action is required to be exhibited, in a moderate amount (approximately 0.001 to 0.1 $g/cm^2$), by an arbitrary method known in the field (for example, application). The topical composition may be regularly used several times a day (for example, approximately 1 to 5 times a day), or may be irregularly used at a timing when an antiperspirant action or an antibacterial action is desired to be exhibited. A period of use is not particularly limited, and for example, several days (for example, approximately 3 days) to approximately 3 months, and preferably approximately 1 week to 1 month.

The pH of the topical composition can be suitably set in accordance with the type of component (A) or component (B), the type and content of other blending components, a dosage form, a usage, and the like. The pH of the topical composition is not limited insofar as the pH is in a range that can be physiologically or pharmaceutically allowed, and for example, can be 2 to 9, preferably approximately 3 to 8, and more preferably approximately 5 to 7, from the viewpoint of safety such as skin stimulation.

A container to be filled with the topical composition is not particularly limited. The container may be containers used for pharmaceutical external preparations, quasi-pharmaceutical products, or cosmetics. Examples of such a container include a container in which a part or all of the surface in contact with the topical composition, preferably all of the surface is configured of at least one material selected from the group consisting of a polyolefin resin, an acrylic acid resin, polyester, polycarbonate, a fluorine resin, polyvinyl chloride, polyamide, an ABS resin, an AS resin, polyacetal, modified polyphenylene ether, polyarylate, polysulfone, polyimide, cellulose acetate, aluminum, and glass.

From the viewpoint of handleability, polyethylene (PE) (including high-density polyethylene (HDPE), low-density polyethylene (LDPE), ultra-low-density polyethylene, linear low-density polyethylene (LLDPE), ultra-high molecular weight polyethylene, and the like); polypropylene (PP) (including isotactic polypropylene, syndiotactic polypropylene, atactic polypropylene, and the like); a polyolefin resin such as an ethylene-propylene copolymer, polymethyl pentene, polybutene-1, and 1,2-polybutadiene; and a polyester resin such as polyethylene terephthalate, polybutylene terephthalate, and polyethylene naphthalate are preferable as the material of the container to be filled with the topical composition. Polyethylene or polypropylene is more preferable.

Although preferred compound names of essential components and arbitrary components that are used in the composition of the invention have been described in the above description, the composition of the invention also includes a composition that is obtained by arbitrarily combining the components, and a composition that is obtained by arbitrarily combining the content (concentration) ranges of each of the components. In addition, the numerical ranges such as the concentration, the pH, and parts by weight can also be arbitrarily combined, and in a case where there are a plurality of numerical ranges, the upper limit values or the lower limit values of each of the numerical ranges can also be arbitrarily combined.

Hereinafter, the composition of the invention will be described in more details by Test Examples and Examples, but the invention is not limited to Examples.

<Test Example 1 Adhesiveness (Film Strength) Evaluation>

The topical composition of the invention was prepared, applied onto one surface of a flexible sheet, and dried to form a film on the sheet. Then, the sheet including the film formed on the surface was gradually bent, and the amount of film peeled off from the sheet was weighed to evaluate the adhesiveness of the film (that is, the adhesiveness of the film increases as the amount of film peeled off from the sheet decreases). More detailed evaluation method will be described below.

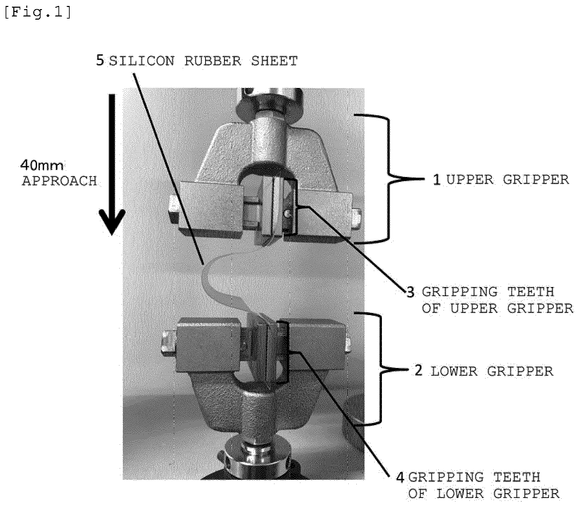

Topical compositions (aqueous solutions) shown in Table 1 were prepared. 1 mL of the aqueous solution was dropped onto the center (a position of a length of 7 cm and a breadth of 3.5 cm) of a rectangular silicon rubber sheet having a thickness of 0.1 mm, a length of 14 cm, and a breadth of 7 cm and dried at 25°C and humidity of 50±5% for 2 days. After that, an upper end and a lower end of the silicon rubber sheet were vertically fixed to AUTOGRAPH AGS-X 5kN (manufactured by Shimadzu Corporation) provided with a screw flat gripper. At this time, the top and the bottom of the silicon rubber sheet were fixed such that an upper surface of a gripping teeth of an upper gripper and the upper end of the silicon rubber sheet were in the same position and a lower surface of a gripping teeth of a lower gripper and the lower end of the silicon rubber sheet were in the same position. In addition, the right and the left ends of the silicon rubber sheet were fixed such that the center of each of the gripping teeth and the center of the silicon rubber sheet were aligned. After that, a compression test was started (that is, the upper gripper was allowed to approach the lower gripper). A compression (approach) rate was 100 mm/minute, and the test was performed until the stroke of the upper gripper was 40 mm. Immediately after the operation was started (shorter than 1 second after the start), a film-formed surface of the silicon rubber sheet was guided to be recessed in parallel with a horizontal direction of the silicon rubber sheet (the position to which the formulation was applied was guided to be recessed into the shape of a concave by gripping the edge of the center portion of the silicon rubber sheet (that is, a position of length 7 cm)). The silicon rubber sheet was bent in accordance with the compression (refer to Fig. 1). At a time point when the stroke was 40 mm, the sheet was folded to an unrestorable state. A weight change of the film adhering to the silicon rubber sheet before and after the compression test was measured, and a weight decrease rate of the film was calculated in accordance with Equality 1. In a case where the formed film is vulnerable to the bending, that is, has low adhesiveness, a part or all of the film was peeled off from the silicon rubber sheet in the middle of the compression, and thus, the weight decrease rate increases. In addition, the film that is vulnerable to the bending and has low adhesiveness is likely to be peeled off when the stroke is small. The weight decrease rate when the film adhered tightly to the silicon rubber sheet and was not peeled off from the silicon rubber sheet at all was evaluated as "0". Results are summarized in Table 1. Note that, the topical composition that was not solidified by drying and dripped from the silicon rubber sheet was evaluated as unmeasurable "(-)".

Equality 1:

$$\text{Weight Decrease Rate (\%)} = \frac{(S_1 - S_0) - (S_2 - S_0)}{S_1 - S_0} \times 100$$

In the equality, $S_0$ indicates the weight of the silicon rubber sheet itself, $S_1$ indicates a weight measured after dropping the topical composition onto the silicon rubber sheet and drying the topical composition (before the compression test), and $S_2$ indicates a weight measured after the compression test.

Table 1. Bending Test

| Component name (wt.%) | Comparative Example 1-1 | Comparative Example 1-2 | Comparative Example 1-3 | Comparative Example 1-4 | Example 1-1 | Example 1-2 | Example 1-3 | Example 1-4 | Example 1-5 | Example 1-6 | Example 1-7 | Example 1-8 | Example 1-9 | Example 1-10 |
|---|---|---|---|---|---|---|---|---|---|---|---|---|---|---|
| Xylitol (MW: 152) | | | | | | | | 0.8 | 5 | 10 | 10 | | | |
| Erythritol (MW: 122) | 10 | | | | 10 | 10 | 5 | | | | | 8 | 1 | 1 |
| Glycerin (MW: 92) | | | 10 | | | | | | | | | | | |
| Zinc paraphenol sulfonate | | | | 3 | | | | | | | | 3 | 3 | 3 |
| Aluminum chlorohydrate | | 10 | 10 | | 10 | 15 | 15 | 1 | 15 | 10 | 15 | | | |
| Purified water | balance | balance | balance | balance | balance | balance | balance | balance | balance | balance | balance | balance | balance | balance |
| pH | 6.5 | 4.2 | 4.1 | 5.1 | 4.2 | 4.1 | 4.1 | 4.7 | 4.0 | 4.1 | 4.0 | 5.2 | 5.3 | 5.2 |
| Weight decrease rate (%) | 87 | 93 | - | 80 | 0 | 0 | 0 | 0 | 0 | 0 | 0 | 0 | 0 | 0 |

MW: molecular weight

The numerical values of each of the components in Table 1 are represented by wt.%, the total of a "balance" of purified water and the numerical values of other components is 100 (wt.%) (for example, the balance of purified water in Example 1-1 is 80).

As shown in Table 1, in a case of a topical composition containing only polyhydric alcohol having a molecular weight of 100 to 200 (Comparative Example 1-1) or a topical composition containing only a non-polymerizable cationic compound (Comparative Example 1-2 and Comparative Example 1-4), the strength of the film with respect to the bending was small, and strength considered to be sufficient to adhere to the skin was not obtained. Without intending to be bound by any theory, it is considered that this is caused by high crystallizability of polyhydric alcohol or a metal salt, which is a solid at a normal temperature. In addition, a topical composition containing glycerin and aluminum chlorohydrate (Comparative Example 1-3) did not form a film, and the topical composition dripped from the silicon rubber sheet. Without intending to be bound by any theory, it is considered that this is caused by the fact that glycerin is liquid at a normal temperature (a molecular weight is small). In Comparative Example 1-1, when the stroke was approximately 31 mm, most of the film was peeled off. In Comparative Example 1-2, when the stroke was approximately 13 mm, most of the film was peeled off. In Comparative Example 1-4, when the stroke was approximately 28 mm, most of the film was peeled off.

On the other hand, in a case of a topical composition containing both of polyhydric alcohol having a molecular weight of 100 to 200 and a non-polymerizable cationic compound (Examples 1-1 to 1-10), even after the silicon rubber sheet was folded, the film adhered onto sheet, and the weight decrease rate was 0%. From this, it was confirmed that the adhesiveness of the film is improved (the strength of the film with respect to the bending increases), and the film is less likely to be peeled off even in a case of being physically stimulated, by using the polyhydric alcohol having a molecular weight of 100 to 200 and the non-polymerizable cationic compound together.

<Test Example 2 Astringency Evaluation>

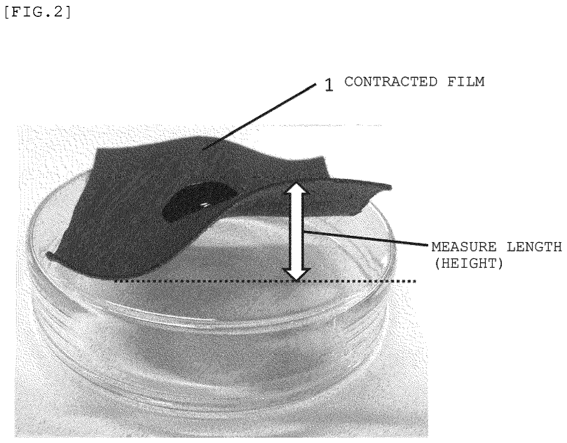

Topical compositions shown in Table 2 were prepared. Subsequently, 1 ml of each composition was dropped onto the center of an artificial leather (manufactured by Idemitsu Techno Fine Co., Ltd., Product Number: PBZ13001, black) of 5 cm × 5 cm, which was contained in a glass petri dish having a diameter of 7 cm, and dried overnight at room temperature without a lid. In a case where a composition has astringency, the artificial leather is contracted as the composition is dried. After a lapse of one night, as illustrated in Fig. 2 (photographed by placing the artificial leather on the transparent petri dish, in order to be easily viewable), for the corrugation of the artificial leather caused by the contraction, a height from a horizontal surface of the most corrugated portion (the highest portion with respect to the horizontal surface) in each of the artificial leathers was measured, the degree of improvement thereof was calculated in accordance with Equality 2, and comparison was performed. The astringence is suppressed as the degree of astringence improvement increases. Results are summarized in Table 2. Note that, a case where there was no astringence was evaluated as the degree of astringence improvement of 100%. Here, Examples 2-1 and 2-2 were calculated with respect to Comparative Example 2-1, and Examples 2-3 to 2-6 were calculated with respect to Comparative Example 2-2.

Equality 2:

$$\text{Degree of Astringence Improvement (\%)} = \frac{\text{Height of Comparative Example} - \text{Height of Example}}{\text{Height of Comparative Example}} \times 100$$

Table 2. Astringence Test

| Component name (wt. %) | Comparative Example 2-1 | Example 2-1 | Example 2-2 | Comparative Example 2-2 | Example 2-3 | Example 2-4 | Example 2-5 | Example 2-6 |
|---|---|---|---|---|---|---|---|---|
| Xylitol (MW: 152) | | | 10 | | | | 5 | 10 |
| Erythritol (MW: 122) | | 10 | | | 5 | 10 | | |
| Aluminum chlorohydrate | 10 | 10 | 10 | 15 | 15 | 15 | 15 | 15 |
| Purified water | balance | balance | balance | balance | balance | balance | balance | balance |
| pH | 4.2 | 4.2 | 4.1 | 4.1 | 4.1 | 4.1 | 4.0 | 4.0 |
| Degree of astringence improvement (%) | - | 73 | 100 | - | 71 | 76 | 71 | 100 |

In a composition containing only aluminum chlorohydrate (Comparative Example 2-1 and Comparative Example 2-2), the artificial leather was greatly corrugated, and strong astringence was observed. In contrast, in a composition further containing polyhydric alcohol having a molecular weight of 100 to 200 (Examples 2-1 to 2-6), it was observed that the corrugation was small, and the astringence was weak. That is, it was possible to decrease the astringency of aluminum chlorohydrate by using aluminum chlorohydrate and the polyhydric alcohol having a molecular weight of 100 to 200 together.

Aluminum chlorohydrate, an antiperspirant component, may cause a user to feel skin stimulation (tenseness, tingling, itchiness, and the like) due to an astringence action thereof. Such an astringence action does not correlate with an antiperspirant action, and even in a case where there is no astringence action, the antiperspirant action can be obtained. Accordingly, it is preferable that the astringence action that can be felt as skin stimulation is low. In this test, unexpectedly, it was confirmed that the astringence action of the antiperspirant component is suppressed by the polyhydric alcohol having a molecular weight of 100 to 200. Therefore, according to the invention, it is possible to decrease the skin stimulation due to the antiperspirant component with astringency.

[Formulation Example]

Hereinafter, Formulation Examples of the topical compositions will be described (% indicates wt.%). The topical compositions can be produced in accordance with an ordinary method in the form thereof.

Formulation Example 1: Antiperspirant (Cream)

Benzalkonium Chloride 0.07%
Isopropyl Methyl Phenol 0.07%
Aluminum chlorohydrate 16%
Cyclopentasiloxane 25%
Alkyl Polyacrylate 6%
POE-POP Dimethicone Copolymer 3.5%
Concentrated Glycerin 7%
Isononanoate Isononyl 5%
1-Menthol 0.5%
Erythritol 10%
Purified Water balance
Total 100%

Formulation Example 2: Antiperspirant (Gel)

Benzalkonium Chloride 0.06%
Isopropyl Methyl Phenol 0.05%
Aluminum chlorohydrate 8%
Magnesium Aluminometasilicate 0.5%
Dipropylene Glycol 10%
Polyoxyethylene Glycol 0.5%
Hydroxypropyl Methyl Cellulose 0.5%
Xanthane Gum 0.5%
Ethanol 10%
1-Menthol 0.3%
1-Menthyl Glyceryl Ether 0.3%
Mint Oil 0.3%
Fragrance 0.3%
Xylitol 15%
Purified Water balance
Total 100%

Formulation Example 3: Antiperspirant (Roll-on Type)

Benzalkonium Chloride 0.06%
Isopropyl Methyl Phenol 0.06%
Zinc Para-Phenolsulfonate 5%

Butylene Glycol 5%
Talc 3%
Nylon Powder 0.5%
Cetyl Trimethyl Ammonium Bromide Liquid 1%
Hydrophobized Hydroxypropyl Methyl Cellulose 0.1%
Dehydrated Ethanol 40%
Erythritol 4%
Xylitol 4%
Purified Water balance
Total 100%

Formulation Example 4: Antiperspirant (Mist)

Benzalkonium Chloride 0.05%
Isopropyl Methyl Phenol 0.06%
Zinc Para-Phenolsulfonate 1%
Butylene Glycol 3%
Aluminum chlorohydrate 0.05%
Sericite Complex 0.2%
1-Menthol 0.5%
Fragrance 0.05%
Dehydrated Ethanol 60%
Sorbitol 1%
Purified Water balance
Total 100%

Formulation Example 5: Antiperspirant (Stick)

Benzalkonium Chloride 0.055%
Isopropyl Methyl Phenol 0.05%
Aluminum chlorohydrate 5%
Polyethylene Wax 5%
Microcrystalline Wax 5%
Glyceryl Monostearate 3%
Octyl Dodecanol 5%
Talc 10%
Methyl Polysiloxane 5%
Decamethyl Pentasiloxane 35%
Silylated Silicic Anhydride 1%
Hydroxyapatite 0.3%
Cetyl trimethyl ammonium Bromide Solution 0.5%
Zinc Oxide 0.3%
Fragrance 0.1%
Cetyl Alcohol 4%
Stearyl Alcohol 4%
Erythritol 1%
Purified Water balance
Total 100%

Formulation Example 6: Antiperspirant (Sheet)

Aluminum chlorohydrate 1%
Polyoxyethylene Polyoxypropylene Decyl Tetradecyl Ether 1%
Methyl Siloxane Reticular Copolymer 0.2%
Dehydrated Ethanol 25%
Paraben 0.1%
Erythritol 0.5%
Fragrance 0.02%

pH Adjuster moderate amount
Purified Water balance
Total 100%

INDUSTRIAL APPLICABILITY

According to the invention, even in a case of not using a polymer, a topical composition having high adhesiveness with respect to the skin can be attained. In particular, the topical composition is suitable for being used as an antiperspirant by using the antiperspirant component as the component (B).

**Claims**

1.   A topical composition, comprising:

(A) at least one polyhydric alcohol having a molecular weight of 100 to 200; and
(B) at least one non-polymerizable cationic compound.

2.   The topical composition according to claim 1,
wherein the number of hydroxy groups of the component (A) is 4 or more.

3.   The topical composition according to claim 1 or 2,
wherein the component (B) is one or more types selected from the group consisting of a compound containing a metal to be a divalent or higher cation in an aqueous solution, a basic amino acid, alkanol amine, and alkyl amine.

4.   The topical composition according to any one of claims 1 to 3,
wherein the component (A) is one or more types selected from the group consisting of glucose, sorbitol, fructose, xylitol, and erythritol.

5.   The topical composition according to any one of claims 1 to 4,
wherein the component (B) is one or more types selected from the group consisting of zinc para-phenolsulfonate, aluminum chlorohydrate, alum, arginine, and lysine.

6.   The topical composition according to any one of claims 1 to 5,
wherein a concentration of the component (A) is 4 to 20 wt.%.

7.   The topical composition according to any one of claims 1 to 6,
wherein the topical composition is an antiperspirant composition.

8.   The topical composition according to any one of claims 1 to 7,
wherein when a total amount of the component (A) is set to 1 part by weight, a total amount of the component (B) is 0.01 to 25 parts by weight.

9.   The topical composition according to claim 1,
wherein the topical composition is an antiperspirant composition, comprising:

(A) 1 to 20 wt.% of at least one polyhydric alcohol selected from the group consisting of xylitol and erythritol; and
(B) 1 to 20 wt.% of at least one non-polymerizable cationic compound selected from the group consisting of zinc para-phenolsulfonate, aluminum chlorohydrate, and alum.

10.   The topical composition according to claim 1,
wherein the topical composition is an antiperspirant composition, comprising:

(A) 1 to 15 wt.% of erythritol; and
(B) 1 to 20 wt.% of at least one non-polymerizable cationic compound selected from the group consisting of zinc para-phenolsulfonate and aluminum chlorohydrate, and

when a total amount of the component (A) is set to 1 part by weight, a total amount of the component (B) is in a range of 1 to 3 parts by weight.

11. The topical composition according to any one of claims 1 to 10,
wherein the topical composition has film-forming ability.

12. Use of (A) at least one polyhydric alcohol having a molecular weight of 100 to 200 for reacting with (B) at least one non-polymerizable cationic compound to form a film that adheres to skin.

[Fig.1]

5 SILICON RUBBER SHEET

40mm
APPROACH

1 UPPER GRIPPER

3 GRIPPING TEETH
OF UPPER GRIPPER

2 LOWER GRIPPER

4 GRIPPING TEETH
OF LOWER GRIPPER

[FIG.2]

1 CONTRACTED FILM

MEASURE LENGTH (HEIGHT)

<div style="text-align:center">**INTERNATIONAL SEARCH REPORT**</div>

| International application No. |
| --- |
| PCT/JP2020/032897 |

**A. CLASSIFICATION OF SUBJECT MATTER**
A61K 8/34(2006.01)i; A61K 8/26(2006.01)i; A61K 8/41(2006.01)i; A61K 8/44(2006.01)i; A61K 8/46(2006.01)i; A61K 8/60(2006.01)i; A61K 47/02(2006.01)i; A61K 47/10(2006.01)i; A61K 47/18(2006.01)i; A61K 47/20(2006.01)i; A61K 47/26(2006.01)i; A61Q 15/00(2006.01)i
FI:     A61K8/34; A61K8/26; A61K8/41; A61K8/44; A61K8/46; A61K8/60; A61K47/02; A61K47/10; A61K47/18; A61K47/20; A61K47/26; A61Q15/00

According to International Patent Classification (IPC) or to both national classification and IPC

**B. FIELDS SEARCHED**

Minimum documentation searched (classification system followed by classification symbols)
A61K8/34; A61K8/26; A61K8/41; A61K8/44; A61K8/46; A61K8/60; A61K47/02; A61K47/10; A61K47/18; A61K47/20; A61K47/26; A61Q15/00

Documentation searched other than minimum documentation to the extent that such documents are included in the fields searched
| | |
| --- | --- |
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
Mintel GNPD

**C. DOCUMENTS CONSIDERED TO BE RELEVANT**

| Category* | Citation of document, with indication, where appropriate, of the relevant passages | Relevant to claim No. |
| --- | --- | --- |
| X | JP 2004-285052 A (LION CORP.) 14 October 2004 (2004-10-14) claims, paragraphs [0010], [0014], [0025], [0028], [0057] | 1–12 |
| X | JP 2005-218745 A (OJI NEPIA CO., LTD.) 18 August 2005 (2005-08-18) claims, paragraphs [0025], [0029]–[0030], [0035] | 1–12 |
| X | JP 2005-179803 A (TAIMEI CHEMICALS CO., LTD.) 07 July 2005 (2005-07-07) claims, paragraphs [0012]– [0013], [0020]–[0021], [0032] | 1–9, 11–12 |
| X | JP 2001-316215 A (TAIMEI CHEMICALS CO., LTD.) 13 November 2001 (2001-11-13) claims, paragraphs [0014]–[0018], [0028], [0035] | 1–9, 11–12 |

☒ Further documents are listed in the continuation of Box C.  ☒ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 08 October 2020 (08.10.2020) | 02 November 2020 (02.11.2020) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| | International application No. |
|---|---|
| | PCT/JP2020/032897 |

C (Continuation). DOCUMENTS CONSIDERED TO BE RELEVANT

| Category* | Citation of document, with indication, where appropriate, of the relevant passages | Relevant to claim No. |
|---|---|---|
| X | LION CORPORATION, "Deodorant Powder Spray, Mintel GNPD" [Online], February 2010 [retrieval date 08 October 2020], ineternet:<URL:https://www.mintel.com>, Accession no. 1289044 in particular, product description, content of the appeal, component | 1-12 |
| X | LUMENE, "Antiperspirant Deodorant Roll-On, Mintel GNPD" [online], June 2008 [retrieval date 08 October 2020], internet:<URL:https://www.mintel.com>, accession no. 934350 in particular, product description, content of the appeal, component | 1-9, 11-12 |
| X | SK PLANET, "Deo Stick, Mintel GNPD" [online], August 2018 [retrieval date 08 October 2020], internet:<URL:https://www.mintel.com>, Accession no. 5870363 in particular, product description, content of the appeal, component | 1-9, 11-12 |
| X | BEAUTE PRESTIGE INTERNATIONAL, "Roll-On Deodorant, Mintel GNPD" [online], March 2010 [retrieval date 08 October 2020], internet:<URL: https://www.mintel.com>, Accession no. 1292651 in particular, product description, content of the appeal, component | 1-9, 11-12 |
| X | COLGATE-PALMOLIVE, "Deodorant, Mintel GNPD" [online], March 2010 [retrieval date 08 October 2020], internet:<URL: https://www.mintel.com>, Accession no. 1287751 in particular, product description, content of the appeal, component | 1-9, 11-12 |
| X | MENG XIANG MARKETING, "Pore Treatment Mask, Mintel GNPD" [online], December 2018 [retrieval date 08 October 2020], internet:<URL: https://www.mintel.com>, Accession no.6183943 in particular, product description, content of the appeal, component | 1-6, 8, 11-12 |

Form PCT/ISA/210 (continuation of second sheet) (January 2015)

**INTERNATIONAL SEARCH REPORT**
Information on patent family members

International application No.

PCT/JP2020/032897

| Patent Documents referred in the Report | Publication Date | Patent Family | Publication Date |
|---|---|---|---|
| JP 2004-285052 A | 14 Oct. 2004 | (Family: none) | |
| JP 2005-218745 A | 18 Aug. 2005 | (Family: none) | |
| JP 2005-179803 A | 07 Jul. 2005 | (Family: none) | |
| JP 2001-316215 A | 13 Nov. 2001 | (Family: none) | |

Form PCT/ISA/210 (patent family annex) (January 2015)

22

**EP 4 026 534 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004059501 A
- JP 2018203683 A